# EUROPÄISCHE PATENTSCHRIFT

(11) **EP 1 567 073 B1**
(45) Veröffentlichungstag und Bekanntmachung des Hinweises auf die Patenterteilung: **02.04.2014**
(21) Anmeldenummer: 02808207.1
(22) Anmeldetag: 06.12.2002
(51) Int. Cl.: A61B 17/70, A61B 17/64

(54) **VORRICHTUNG ZUR STABILISIERUNG VON KNOCHEN**
DEVICE FOR STABILISING BONES
DISPOSITIF POUR STABILISER DES OS

(43) Veröffentlichungstag der Anmeldung: 31.08.2005
(73) Patentinhaber: Synthes GmbH, 4436 Oberdorf (CH)
(72) Erfinder: SCHLÄPFER, Fridolin, CH-4434 Hölstein (CH); HESS, Martin, CH-4434 Hölstein (CH)
(74) Vertreter: Lusuardi, Werther
(86) Internationale Anmeldenummer: PCT/CH2002/000672
(87) Internationale Veröffentlichungsnummer: WO 2004/052218

(56) Entgegenhaltungen:
- EP-A- 1 072 228
- WO-A-94/00066
- DE-A- 4 040 368
- FR-A- 2 715 057
- US-B1- 6 273 914

## Beschreibung

Die Erfindung bezieht sich auf eine Vorrichtung zur schwenkbaren Verbindung von zwei zu einer Fixationseinrichtung für Knochen zählenden Stabilisationsteilen mit einem Knochenfixationsmittel, gemäss dem Oberbegriff des Patentanspruchs 1.

Eine Vorrichtung gemäß dem Oberbegriff des Anspruchs 1 ist aus der US 6,273,914 B1 bekannt.

Eine osteosynthetische Fixationsvorrichtung zur Verbindung eines Fixationselementes, beispielsweise einer Pedikelschraube mit einem Längsträger ist aus der WO 94/00066 SCHLÄPFER bekannt. Diese bekannte Fixationsvorrichtung eignet sich als Platten/Schrauben-System, als Fixateur externe oder interne, sowie insbesondere zur Wirbelsäulenfixation. Sie weist eine Gelenkverbindung zwischen dem Fixationselement und dem Längsträger auf, welche ein Verbindungselement mit einem durchgehend offenen, sphärisch ausgestalteten Hohlraum, und ein elastisch spreizbares Klemmelement mit einer zur Hohlraumwand komplementär sphärischen Aussenwand umfasst. Das Fixationselement hat ein konisches Segment, welches an das in den Knochen schraubbare Verankerungssegment angrenzt. Das konische Segment ist in einem Innenkonus im Klemmelement verkeilbar, wodurch das Klemmelement radial gespreizt und im Hohlraum verklemmt wird. Die Aufnahme für den Längsträger im Verbindungselement ist durch eine Bohrung mit quer zur Zentralachse des Fixationselementes verlaufender Bohrungsachse realisiert. Die in der WO 94/00066 SCHLÄPFER beschriebene Vorrichtung erlaubt nur die Verbindung zweier Körper. Sobald drei und mehr Körper verbunden werden müssen, versagt das System.

Hier will die Erfindung Abhilfe schaffen. Der Erfindung liegt die Aufgabe zugrunde, eine Verbindung zwischen einem Knochenfixationsmittel und zweier Längsträger herzustellen, wobei die Winkel der zwei Längsträger zum Knochenfixationsmittel individuell eingestellt und fixiert werden können. Damit ist es möglich, sequentiell von einem Körper zu einem anderen Körper ein multisegmentales Fixationssystem aufzubauen.

Die Erfindung löst die gestellte Aufgabe mit einer Vorrichtung zur schwenkbaren Verbindung von zwei zu einer Fixationseinrichtung für Knochen zählenden Stabilisationsteilen mit einem Knochenfixationsmittel, welche die Merkmale des Anspruchs 1 aufweist.

Die durch die Erfindung erreichten Vorteile sind im wesentlichen darin zu sehen, dass dank der erfindungsgemässen Vorrichtung zwei Stabilisationsteile, beispielsweise im Falle einer Wirbelsäulenfixationseinrichtung zwei Längsträger unabhängig voneinander relativ zu einem Knochenfixationsmittel, beispielsweise einer Pedikelschraube oder einem Pedikelhaken schwenkbar verbindbar sind. Dadurch erübrigt sich ein aufwendiges Vorbiegen des Längsträgers.

In einer bevorzugten Ausführungsform umfasst die Vorrichtung zusätzlich am Kopfteil angreifende Spannmittel zur axialen Verschiebung des mindestens einen Keils oder Konus.

In weiteren Ausführungsformen weist die Vorrichtung folgende Merkmale auf:
- mindestens ein Keil oder Konus ist einstückig mit dem Kopfteil des Knochenfixationsmittels; und/oder
- mindestens ein Keil oder Konus ist einstückig mit dem Spannmittel.

In wiederum einer weiteren Ausführungsform umfasst die Vorrichtung einen unteren und einen oberen Keil oder Konus, wobei sich der untere Keil oder Konus gegen den Verankerungsteil am Knochenfixationsmittel erweitert und sich der obere Keil oder Konus gegen das hintere Ende des Knochenfixationsmittels erweitert.

Bei einer Ausführungsform mit zwei Keilen oder Koni sind beispielsweise folgende Anordnungen möglich:
- der obere Keil oder Konus ist einstückig mit dem Spannmittel;
- der untere Keil oder Konus ist einstückig mit dem Kopfteil des Knochenfixationsmittels.

In einer anderen Ausführungsform sind die Knochenfixationsmittel derart ausgestaltet, dass ihr Kopfteil endständig einen zur Zentralachse koaxialen Bolzen mit einem Aussengewinde umfasst. Die Spannmittel bestehen vorzugsweise aus einer Mutter, welche über das Aussengewinde am Kopfteil des Knochenfixationsmittels schraubbar ist.

In wiederum einer anderen Ausführungsform sind die Knochenfixationsmittel derart ausgestaltet, dass ihr Kopfteil an seinem hinteren Ende eine zur Zentralachse koaxiale Bohrung mit einem Innengewinde aufweist. Die Spannmittel bestehen hier vorzugsweise aus einer Schraube, welche in das Innengewinde schraubbar ist.

In einer weiteren Ausführungsform der erfindungsgemässen Vorrichtung sind die Kavitäten in den Stabilisationsteilen sphärisch und die Aussenwände der Klemmelemente dazu komplementär sphärisch ausgestaltet.

In einer anderen Ausführungsform der erfindungsgemässen Vorrichtung sind die Aussenwände der Klemmelemente sphärisch ausgestaltet während die Kavitäten in den Stabilisationsteilen einen gegenüber den Klemmelementen vergrösserten zentralen Hohlraum mit zwei endständigen Verengungen aufweisen. Die Verengungen haben endständig zur Zentralachse konzentrische, kreisförmige Kanten, woran die Klemmelemente anliegen. Durch diese Ausgestaltung der Kavitäten ist zwischen den Klemmelementen und den Kavitäten ein linienförmiger Kontakt herstellbar.

In wiederum einer anderen Ausführungsform der erfindungsgemässen Vorrichtung sind die Kavitäten sphärisch ausgestaltet währende die Aussenwände der Klemmelemente mit axial hintereinander angeordneten kreiszylindrischen Segmenten ausgestaltet sind. Auch durch diese Ausgestaltung der Klemmelementen ist zwischen den Klemmelementen und den Kavitäten ein linienförmiger Kontakt herstellbar.

In einer weiteren Ausführungsform ist das im unteren Stabilisationsteil angeordnete Klemmelement durch erste Spannmittel in der Kavität des unteren Stabilisationsteiles fixierbar und das im oberen Stabilisationsteil angeordnete Klemmelement durch zweite Spannmittel in der Kavität des oberen Stabilisationsteiles fixierbar. Damit ist der Vorteil erreichbar, dass die beiden Stabilisationsteile unabhängig voneinander an dem Kopfteil des Knochenfixationsmittels fixierbar sind. Vorzugsweise umfasst das Kopfteil zwei Konen, welche durch ein Gewindezwischenstück getrennt werden, wobei das Gewindezwischenstück axial gegenüber dem Klemmelement derart positioniert ist, dass das Klemmelement durch ein auf das Gewindezwischenstück aufsetzbares Spannmittel blockierbar ist.

In einer anderen Ausführungsform umfasst ein Spannmittel ein Gewindestück und einen axial angrenzenden, gegen das Gewindestück hin breiter werdenden Konus, welcher komplementär zum Innenkonus in einem der Klemmelemente ausgestaltet ist. Ferner überragt das Klemmelement den Kopfteil axial und das Kopfteil weist am hinteren Ende des Knochenfixationsmittels eine Bohrung mit einem Innengewinde auf, so dass des Gewindestück am Spannmittel in dieses Innengewinde schraubbar ist. Vorzugsweise endet der Konus des Spannmittels an einem das Klemmelement axial überragenden Gewindebolzen, auf dem das mit einem Innengewinde versehene, zweite Spannmittel schraubbar ist.

Umgekehrt lässt sich das erste Spannmittel derart ausgestalten, dass das verjüngte Ende des Konus eine Bohrung mit Innengewinde aufweist, in das ein mit einem Aussengewinde und einem Kopf versehene, zweite Spannmittel eindrehbar ist.

Weitere Ausführungsformen zeichnen sich dadurch aus, dass
- die äusseren Oberflächen der Klemmteile aufgerauht sind;
- die Oberflächen der Kavitäten aufgerauht sind;
- die äusseren Oberflächen der Klemmteile eine dreidimensionale Strukturierung umfassen;
- die Oberflächen der Kavitäten eine dreidimensionale Strukturierung umfassen; und/oder
- dass mindestens ein Klemmelement aus einem Material besteht, welches gegenüber dem Wandmaterial der entsprechenden Kavität weicher ist.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ausführungsform der erfindungsgemässen Vorrichtung mit einem Konus, welcher einstückig mit dem Knochenfixationsmittel ist;
Fig. 2 eine andere Ausführungsform der erfindungsgemässen Vorrichtung mit zwei Konen;
Fig. 3 eine Ausführungsform der erfindungsgemässen Vorrichtung, wobei die Kavitäten zur Aufnahme der Klemmelemente aus mehreren Zylindersegmenten bestehen;
Fig. 4 eine Ausführungsform der erfindungsgemässen Vorrichtung, wobei die Klemmelemente aus mehreren axial aneinandergrenzenden Zylindersegmenten bestehen;
Fig. 5 eine Ausführungsform der erfindungsgemässen Vorrichtung, wobei das obere und das untere Stabilisationsteil Büchsen zur Aufnahme von Längsträgern umfassen; und
Fig. 6 eine Ausführungsform der erfindungsgemässen Vorrichtung, wobei der einstückige Konus der Ausführungsform gemäss Fig. 1 in zwei unabhängige Konen unterteilt ist, mit einem der Konen als Fixiermittel in Verbindung des anderen Konus; und
Fig. 7 eine Ausführungsform der erfindungsgemässen Vorrichtung, wobei der einstückige Konus der Ausführungsform gemäss Fig. 1 durch ein Gewinde unterbrochen ist, um die untere Konusverbindung unabhängig von der oberen Konusverbindung fixieren zu können.

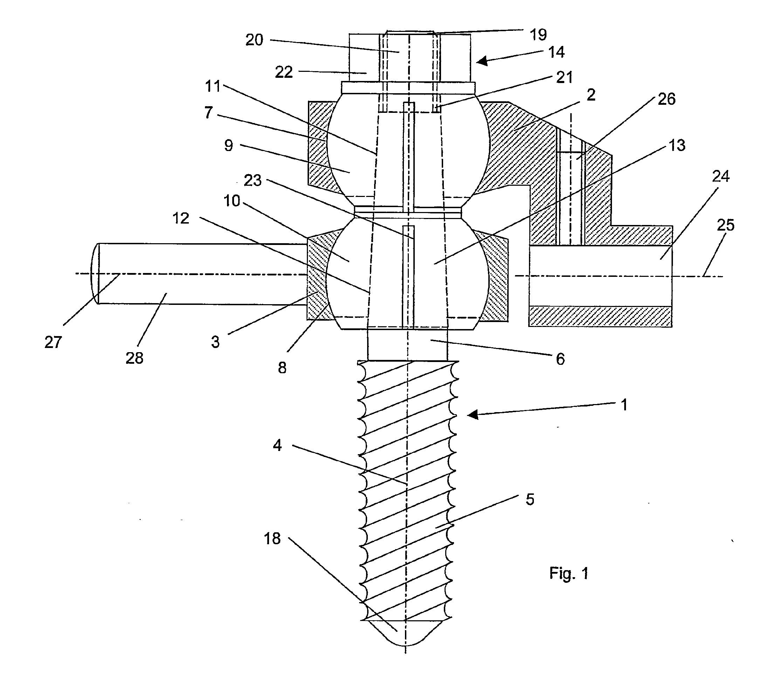

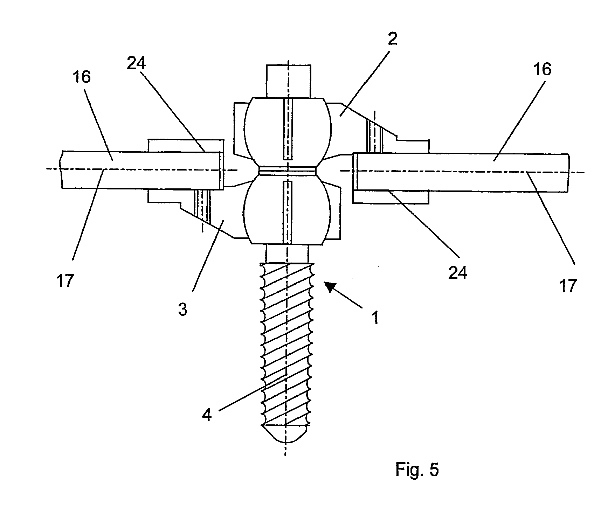

Fig. 1 zeigt eine Ausführungsform der erfindungsgemässen Vorrichtung mit einem als Pedikelschraube ausgestalteten Knochenfixationsmittel 1, einem oberen Stabilisationsteil 2 und einem unteren Stabilisationsteil 3, welche beispielsweise Teile einer Wirbelsäulenfixationsvorrichtung sein können. Beide Stabilisationsteile 2;3 sind gelenkartig mit dem Kopfteil 6 des Knochenfixationsmittels 1 verbunden. Der als Knochenschraube ausgebildete Verankerungsteil 5 des Knochenfixationsmittels 1 ist beispielsweise in einem Pedikel fixierbar. Die Stabilisationsteile 2;3 sind axial hintereinander angeordnet und umfassen je eine sphärische, das jeweilige Stabilisationsteil 2;3 koaxial zur Zentralachse 4 durchdringende Kavität 7;8. In jede Kavität 7;8 ist ein aussen dazu komplementär ausgestaltetes Klemmelement 9;10 mit je einer Zentralbohrung 11;12 eingefügt. Durch die sphärische Ausgestaltung der Kavitäten 7;8 sowie der Klemmelemente 9;10 wird eine kugelgelenkartige Verbindung zwischen jedem Stabilisationsteil 2;3 und dem Knochenfixationsmittel 1 hergestellt. Die Zentralbohrungen 11;12 durchdringen die Klemmelemente 9;10 koaxial zur Zentralachse 4 und sind konisch sich gegen das hintere Ende 19 des Knochenfixationsmittels 1 verjüngend ausgebildet. In die Zentralbohrungen 11;12 eingeführt ist das Kopfteil 6 des Knochenverankerungsmittels 1, welches einen zu beiden Zentralbohrungen 11;12 komplementären, sich gegen das hintere Ende 19 des Knochenfixationsmittels 1 verjüngenden Konus 13 umfasst. Am hinteren Ende 19 des Knochenfixationsmittels 1 ist ein ebenfalls zur Zentralachse 4 koaxialer Bolzen 20 mit einem Aussengewinde 21 angebracht. Als Spannmittel 14 ist eine endständig auf dem ersten Stabilisationsteil 2 aufliegende Mutter 22 über das Aussengewinde 21 schraubbar, so dass beim Anziehen der Mutter 22 die beiden Stabilisationsteile 2;3 axial auf dem Kopfteil 6 verschiebbar und in Richtung des vorderen Endes 18 des Knochenfixationsmittels 1 pressbar sind. Ferner sind die Klemmelemente 9;10 mittels zur Zentralachse 4 parallelen Schlitzen 23 radial, d.h. quer zur Zentralachse 4 elastisch deformierbar ausgestaltet. Dadurch ist erreichbar, dass beim Anziehen der Mutter 22, wenn die beiden konischen Zentralbohrungen 11;12 der Stabilisationsteile 2;3 über den Konus 13 am Kopfteil 5 des Knochenfixationsmittels 1 geschoben werden, eine Keilwirkung zwischen dem Konus 13 und den Wänden der konischen Zentralbohrungen 11;12 auftritt und in der Folge beide Klemmelemente 9;10 quer zur Zentralachse 4 expandiert werden und die sphärischen Aussenwände der Klemmelemente 9; 10 an die sphärischen Wände der Kavitäten 7;8 gepresst werden. Zur Verbindung mit einem weiteren zur Wirbelsäulenfixationsvorrichtung gehörenden Element ist das obere Stabilisationsteil 2 mit einer eine quer zur Zentralachse 4 stehenden Längsachse 25 aufweisenden, aussen am Stabilisationsteil 2 offenen Vertiefung 24 ausgestaltet, so dass beispielsweise ein stabförmiger Längsträger (Fig. 5) in die Vertiefung 24 einführbar und mittels einer senkrecht zur Längsachse 25 in das obere Stabilisationsteil 2 einschraubbaren Arretierschraube 26 lösbar fixierbar ist. Das untere Stabilisationsteil 3 ist mit einem stabförmigen, eine zweite, ebenfalls quer Zentralachse 4 stehenden Längsachse 27 aufweisenden Fortsatz 28 versehen, wobei der Fortsatz 28 und die Vertiefung 24 komplementär ausgestaltet sind, so dass anstelle eines Längsträgers auch ein stabförmiger Fortsatz 28 in die Vertiefung 24 des oberen Stabilisationsteiles 2 einer weiteren Vorrichtung einführbar ist. Die Vertiefung 24 und der stabförmige Fortsatz 28 sind so an den Stabilisationsteilen 2;3 angeordnet, dass die beiden Längsachsen 25;27 in der Ausgangslage der Vorrichtung, d.h. bei nicht rotierten Gelenken koaxial sind.

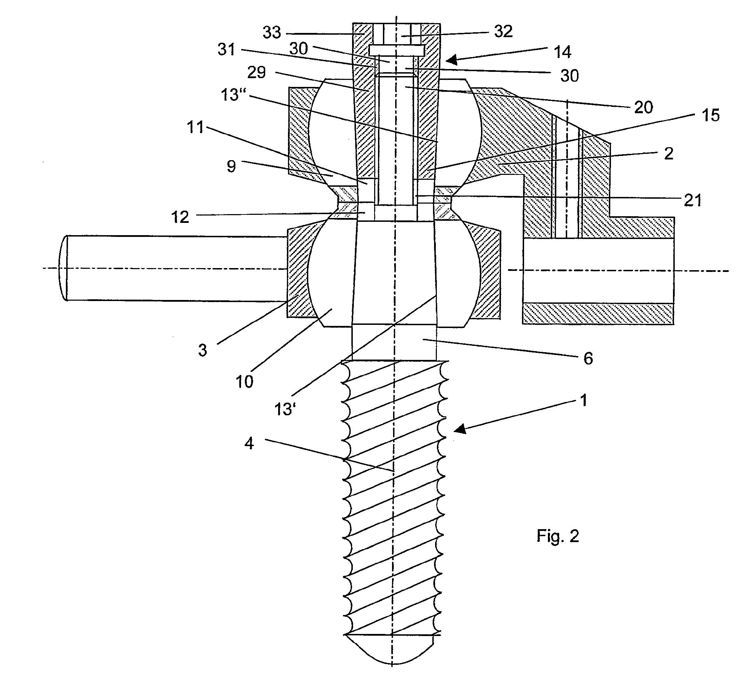

Die in Fig. 2 dargestellte Ausführungsform der erfindungsgemässen Vorrichtung unterscheidet sich von der in Fig. 1 dargestellten Ausführungsform nur darin, dass der Konus 13' am Kopfteil 6 des Knochenfixationsmittels 1 nur das Klemmelement 10 im unteren Stabilisationsteil 3 durchdringt und anschliessend der endständig am Kopfteil 6 des Knochenfixationsmittels 1 angeordnete Bolzen 20 das Klemmelement 9 im oberen Stabilisationsteil 2 durchdringt. Ferner ist das Spannmittel 14 als Hülse 29 mit einer Zentralbohrung 30 ausgestaltet, wobei das in der Zentralbohrung 30 angebrachte Innengewinde 31 über das Aussengewinde 21 am Bolzen 20 schraubbar ist und weist aussen einen zweiten Konus 13" auf, welcher sich gegen das vordere Ende 15 des Spannmittels 14 verjüngt. Die Zentralbohrungen 11;12 in den Klemmelementen 9;15 sind komplementär konisch ausgestaltet, so dass sich die beiden Konusse 13';13" gegen die sich berührenden Enden der Klemmelemente 9;10 verjüngen. Zum Anziehen der Spannmittel 14 sind in der Zentralbohrung 30 am hinteren Ende 33 des Spannmittels 14 Mittel 32 zur Aufnahme eines Schraubendrehers angebracht, welche hier als Innensechskant ausgebildet sind.

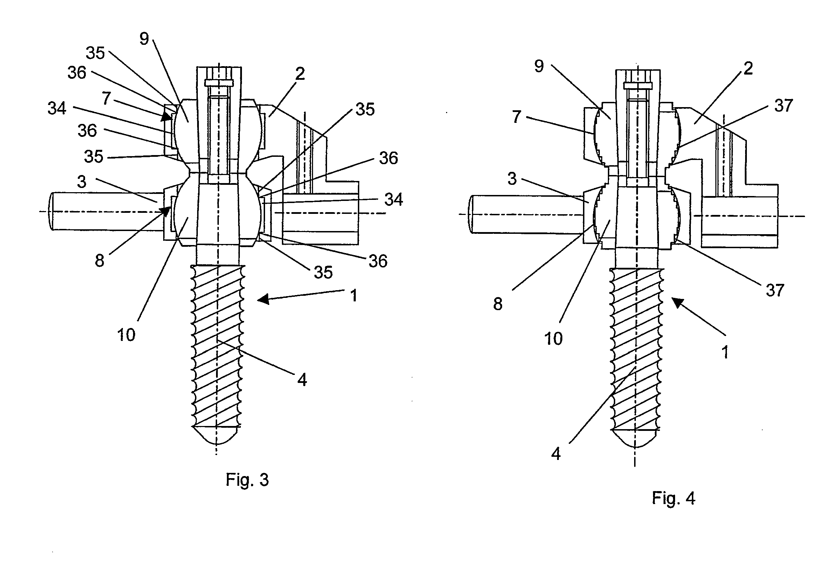

In Fig. 3 ist eine weitere Ausführungsform der erfindungsgemässen Vorrichtung dargestellt, welche sich von der in Fig. 2 dargestellten Ausführungsform nur darin unterscheidet, dass die Kavitäten 7;8 in den beiden Stabilisationsteilen 2;3 mit einem zentralen, gegenüber den Klemmelementen 9; 10 vergrösserten Hohlraum 34 und zwei axial endständigen Verengungen 35 ausgestaltet sind, wobei die Verengungen 35 gegen den Hohlraum 34 gerichtet je eine kreisförmige, zur Zentralachse 4 konzentrische Kante 36 aufweisen, so dass die sphärisch ausgestalteten Klemmelemente 9;10 an den Kanten 36 anliegen und zwischen jeder Kavität 7;8 und dem darin eingefügten Klemmelement 9;10 ein linienförmiger Kontakt hergestellt wird.

In Fig. 4 ist wiederum eine weitere Ausführungsform der erfindungsgemässen Vorrichtung dargestellte, welche sich von der in Fig. 2 dargestellten Ausführungsform nur darin unterscheidet, dass die Klemmelemente 9;10 aussen nicht komplementär zu den sphärischen Kavitäten 7;8 ausgestaltet sind, sondern die sphärische Form der Kavitäten 7;8 durch axial hintereinander angeordnete, kreiszylindrische Segmente 37 an den Klemmelementen 9;10 angenähert werden. Auch damit lässt sich ein linienförmiger Kontakt zwischen den Klemmelementen 9;10 und den Wänden der Kavitäten 7;8 herstellen.

In Fig. 5 ist eine Ausführungsform der erfindungsgemässen Vorrichtung dargestellt, welche sich von der in Fig. 1 dargestellten Ausführungsform nur darin unterscheidet, dass beide Stabilisationsteile 2;3 Vertiefungen 24 zur Aufnahme beispielsweise je eines Längsträgers 16 aufweisen, wobei die Längsachsen 17 zueinander fluchtend und senkrecht zur Zentralachse 4 des Knochenfixationsmittels 1 angeordnet sind. Ferner sind die Stabilisationsteile 2;3 derart ausgestaltet, dass die Längsachsen 17 der Vertiefungen 24 ungefähr an der Grenzfläche zwischen den beiden Stabilisationsteilen 2;3 verlaufen.

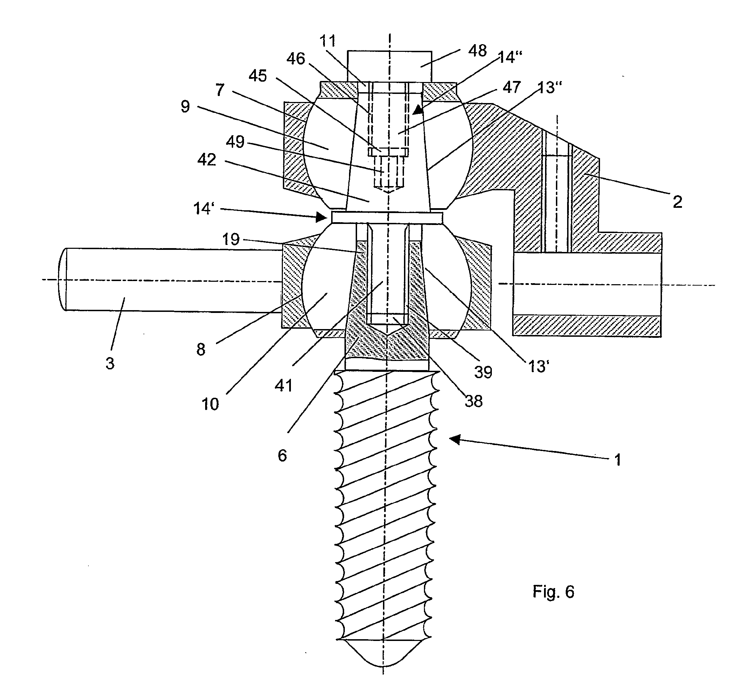

Fig. 6 zeigt eine Ausführungsform der erfindungsgemässen Vorrichtung, welche sich darin von den Ausführungsformen gemäss den Fig. 1 bis 5 unterscheidet, dass das im unteren Stabilisationsteil 3 angeordnete Klemmelement 10 durch erste Spannmittel 14' in der Kavität 8 des unteren Stabilisationsteiles 3 fixierbar ist und das im oberen Stabilisationsteil 2 angeordnete Klemmelement 9 durch zweite Spannmittel 14" in der Kavität 7 des oberen Stabilisationsteiles 2 fixierbar ist. Das Klemmelement 10 überragt den Kopfteil 6 des Knochenfixationsmittels 1 axial, wobei der Kopfteil 6 am hinteren Ende 19 des Knochenfixationsmittels 1 eine Bohrung 38 mit einem Innengewinde 39 aufweist. In dieses Innengewinde 39 einschraubbar ist ein axial endständig am ersten Spannmittel 14' angeordnetes Gewindestück 41, so dass beim Anziehen des ersten Spannmittels 14' mittels eines in die Mittel zur Aufnahme eines Werkzeuges 49 eingefügten Schraubendrehers die Verbindung zwischen dem Kopfteil 6 des Knochenfixationsmittels 1 und dem unteren Stabilisationsteil 3 lösbar blockierbar ist. Ferner umfass des erste Spannmittel 14' einen oben axial an das Gewindestück 41 angrenzenden Konus 42, welcher gegen das Gewindestück 41 hin breiter wird und in den komplementären Konus 13" in der Zentralbohrung 11 im Klemmelement 9 einführbar ist. Das Klemmelement 9 in der Kavität 7 im oberen Stabilisationsteil 2 überragt das verjüngte Ende des am ersten Spannmittel 14' angeordneten Konus 42 überragt das verjüngte Ende des Konus 42 axial. Durch zweite Spannmittel 14", welche als Schraube ausgestaltet sind und einen endständig am Klemmelement 9 anliegenden Kopf 48 sowie ein in die im erste Spannmittel 14' koaxial angebrachte Bohrung 45 mit Innengewinde 45 einschraubbares Aussengewinde 47 umfassen, ist das Klemmelement 9 zusammen mit den ersten Spannmitteln 14' in der Kavität 7 im oberen Stabilisationsteil 2 lösbar blockierbar. Anstelle einer Ausgestaltung der zweiten Spannmittel 14" als Schraube können diese als Mutter ausgestaltet sein und über einen endständig am ersten Spannmittel 14' angebrachten Gewindebolzen schraubbar sein.

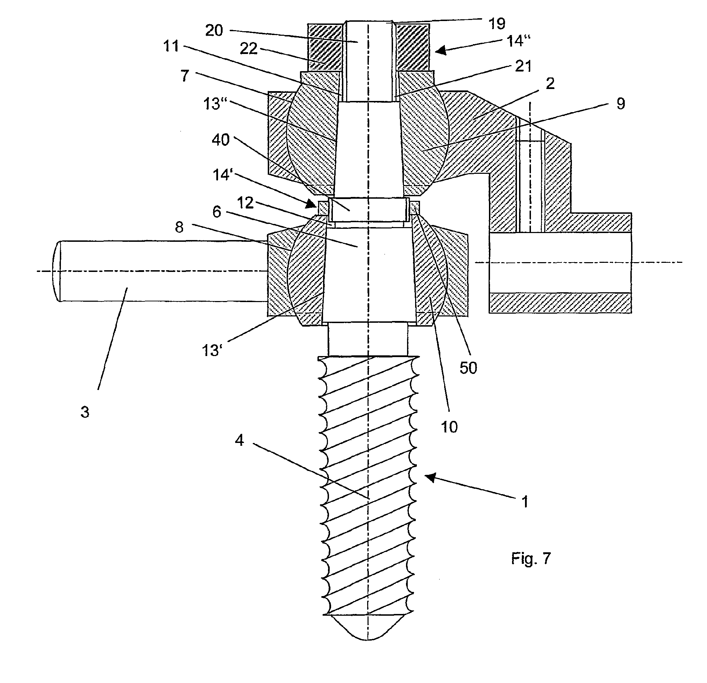

Die in Fig. 7 dargestellte Ausführungsform unterscheidet sich von der in Fig. 1 dargestellten Ausführungsform nur darin, dass der Kopfteil 6 des Knochenfixationsmittel 1 axial hintereinander angeordnet zwei Konen 13'; 13" umfasst, wobei zwischen dem an den Verankerungsteil 5 des Knochenfixationsmittels 1 grenzenden ersten Konus 13' und dem axial endständig angeordneten zweiten Konus 13" ein Gewindezwischenstück 40 angeordnet ist. Dabei wird der erste Konus 13' in die komplementär konisch ausgestaltete Zentralbohrung 12 im Klemmelement 10, welches zur Fixierung des unteren Stabilisationsteiles 3 dient, eingeführt und der zweite Konus 13" in die komplementär konisch ausgestaltete Zentralbohrung 11 im Klemmelement 9, welches zur Fixierung des oberen Stabilisationsteiles 2 dient, eingeführt. Die axiale Position des Gewindezwischenstückes 40 gegenüber dem Klemmelement 10 ist derart gewählt, dass ein als Mutter ausgebildetes, erstes Spannmittel 14' über das Gewindezwischenstück 40 schraubbar ist und auf dem zwischen den Klemmelementen 9;10 liegenden Ende des Klemmelementes 10 zur Anlage bringbar ist. Durch Anziegen des ersten Spannmittels 14' ist somit das untere Stabilisationsteil 3 unabhängig von der Blockierung des oberen Stabilisationsteiles 2 am Kopfteil 6 des Knochenfixationsmittels 1 lösbar blockierbar. Am hinteren Ende 19 des Knochenfixationsmittels 1 ist analog zu der in Fig. 1 dargestellten Ausführungsform ein zur Zentralachse 4 koaxialer Bolzen 20 mit einem Aussengewinde 21 angebracht. Als zweites Spannmittel 14" ist eine endständig auf dem oberen Stabilisationsteil 2 aufliegende Mutter 22 über das Aussengewinde 21 schraubbar, so dass beim Anziehen der Mutter 22 das obere Stabilisationsteil 2 unabhängig von der Blockierung des unteren Stabilisationsteiles 3 am Kopfteil 6 des Knochenfixationsmittels 1 lösbar blockierbar ist.

## Patentansprüche

1. Vorrichtung zur schwenkbaren Verbindung von zwei zu einer Fixationseinrichtung für Knochen gehörenden Stabilisationsteilen (2;3) mit einem Knochenfixationsmittel (1) umfassend mindestens ein Knochenfixationsmittel (1) mit einer Zentralachse (4), welches einen zur Befestigung im oder am Knochen bestimmten Verankerungsteil (5) und einen mit beiden Stabilisationsteilen (2;3) verbindbaren Kopfteil (6) aufweist;
**dadurch gekennzeichnet, dass**
A) die Vorrichtung axial hintereinander angeordnet ein oberes und ein unteres Stabilisationsteil (2;3) mit je einer Kavität (7;8) aufweist, welche das entsprechende Stabilisationsteil (2;3) koaxial zur Zentralachse (4) durchdringt;
B) in jeder Kavität (7;8) rotierbar angeordnet, ein quer zur Zentralachse (4) elastisch deformierbares Klemmelement (9;10) mit einer koaxial zur Zentralachse (4) verlaufenden Zentralbohrung (11;12) vorhanden ist; und
C) die Vorrichtung mindestens einen in den Zentralbohrungen (11;12) axial verklemmbaren Keil oder Konus (13) umfasst, wodurch der Kopfteil (6) des Knochenfixationsmittels (1) in mindestens einem der Stabilisationsteile (2;3) lösbar fixierbar ist.

2. Vorrichtung nach Anspruch 1, **dadurch gekennzeichnet, dass** sie zusätzlich am Kopfteil (6) angreifende Spannmittel (14) zur axialen Verschiebung des mindestens einen Keils oder Konus (13) umfasst.

3. Vorrichtung nach Anspruch 1 oder 2, **dadurch gekennzeichnet, dass** mindestens ein Keil oder Konus (13) einstückig mit dem Kopfteil (6) des Knochenfixationsmittels (1) ist.

4. Vorrichtung nach einem der Ansprüche 1 bis 3, **dadurch gekennzeichnet, dass** mindestens ein Keil oder Konus (13) einstückig mit dem Spannmittel (14) ist.

5. Vorrichtung nach einem der Ansprüche 1 bis 4, **dadurch gekennzeichnet, dass** sie einen unteren und einen oberen Keil oder Konus (13';13") umfasst, wobei sich der untere Keil oder Konus (13') gegen den Verankerungsteil (5) am Knochenfixationsmittel (1) erweitert und sich der obere Keil oder Konus (13") gegen das hintere Ende (19) des Knochenfixationsmittels (1) erweitert.

6. Vorrichtung nach Anspruch 5, **dadurch gekennzeichnet, dass** der obere Keil oder Konus (13") einstückig mit dem Spannmittel (14) ist.

7. Vorrichtung nach Anspruch 5 oder 6, **dadurch gekennzeichnet, dass** der untere Keil oder Konus (13') einstückig mit dem Kopfteil (6) des Knochenfixationsmittels (1) ist.

8. Vorrichtung nach einem der Ansprüche 1 bis 6, **dadurch gekennzeichnet, dass** das Kopfteil (6) des Knochenfixationsmittels (1) endständig einen zur Zentralachse (4) koaxialen Bolzen (20) mit einem Aussengewinde (21) umfasst.

9. Vorrichtung nach Anspruch 8, **dadurch gekennzeichnet, dass** das Spannmittel (14) eine Mutter (22) ist, welche über das Aussengewinde (21) schraubbar ist.

10. Vorrichtung nach einem der Ansprüche 1 bis 6, **dadurch gekennzeichnet, dass** das Kopfteil (6) des Knochenfixationsmittels (1) an seinem hinteren Ende (19) eine zur Zentralachse (4) koaxiale Bohrung mit einem Innengewinde aufweist.

11. Vorrichtung nach Anspruch 10, **dadurch gekennzeichnet, dass** das Spannmittel (14) eine Schraube ist, welche in das Innengewinde schraubbar ist.

12. Vorrichtung nach einem der Ansprüche 1 bis 11, **dadurch gekennzeichnet, dass** ein oberes Klemmelement (9) durch obere Spannmittel (14") und unteres Klemmelement (10) durch untere Spannmittel (14') fixierbar ist.

13. Vorrichtung nach Anspruch 12, **dadurch gekennzeichnet, dass** das Kopfteil (6) zwei Konen (13';13") umfasst, welche durch ein Gewindezwischenstück (40) getrennt werden, wobei das Gewindezwischenstück (40) axial gegenüber dem unteren Klemmelement (10) derart positioniert ist, dass das untere Klemmelement (10) durch ein auf das Gewindezwischenstück (40) aufsetzbares Spannmittel (14') blockierbar ist.

14. Vorrichtung nach Anspruch 12, **dadurch gekennzeichnet, dass**
a) das untere Klemmelement (10) den Kopfteil (6) axial überragt;
b) der Kopfteil (6) am hinteren Ende (19) des Knochenfixationsmittels (1) eine Bohrung (38) mit einem Innengewinde (39) aufweist;
c) das untere Spannmittel (14') ein in das Innengewinde (39) einführbares Gewindestück (41) und einen axial angrenzenden, gegen das Gewindestück (41) hin breiter werdenden Konus (42) umfasst; und
d) der Konus (42) komplementär zum Innenkonus (13") im Klemmelement (9) ist.

15. Vorrichtung nach Anspruch 14, **dadurch gekennzeichnet, dass** der Konus (42) des unteren Spannmittels (14') an einem das obere Klemmelement (9) axial überragenden Gewindebolzen endet, auf dem das mit einem Innengewinde versehene obere Spannmittel (14") schraubbar ist.

16. Vorrichtung nach Anspruch 14, **dadurch gekennzeichnet, dass** das obere Klemmelement (9) das verjüngte Ende des Konus (42) des unteren des Spannmittels (14') axial überragt und das verjüngte Ende des Konus (42) eine Bohrung (45) mit Innengewinde (46) aufweist, in das ein mit einem Aussengewinde (47) und einem Kopf (48) versehene obere Spannmittel (14") eindrehbar ist.

17. Vorrichtung nach einem der Ansprüche 1 bis 16, **dadurch gekennzeichnet, dass** mindestens ein Klemmelement (9;10) aussen aus mehreren axial hintereinander angeordneten kreiszylindrischen Segmenten (37) bestehen.

18. Vorrichtung nach einem der Ansprüche 1 bis 16, **dadurch gekennzeichnet, dass** mindestens eine Kavität (7;8) axial endständig je eine Verengung (35) mit einer zur Zentralachse (4) konzentrischen, kreisförmigen Kante (36) aufweist.

19. Vorrichtung nach einem der Ansprüche 1 bis 16, **dadurch gekennzeichnet, dass** die äusseren Oberflächen der Klemmteile (9;10) aufgerauht sind.

20. Vorrichtung nach einem der Ansprüche 1 bis 16 ober 19, dass die Oberflächen der Kavitäten (7;8) aufgerauht sind.

21. Vorrichtung nach einem der Ansprüche 1 bis 16, **dadurch gekennzeichnet, dass** die äusseren Oberflächen der Klemmteile (9;10) eine dreidimensionale Strukturierung umfassen.

22. Vorrichtung nach einem der Ansprüche 1 bis 16 ober 21, dass die Oberflächen der Kavitäten (7;8) eine dreidimensionale Strukturierung umfassen.

23. Vorrichtung nach einem der Ansprüche 1 bis 22, **dadurch gekennzeichnet, dass** mindestens ein Klemmelement (9;10) aus einem Material besteht, welches gegenüber dem Wandmaterial der entsprechenden Kavität (7;8) weicher ist.

24. Vorrichtung nach einem der Ansprüche 1 bis 23, **dadurch gekennzeichnet, dass** durch den mindestens einen in den Zentralbohrungen (11;12) axial verklemmbaren Keil oder Konus (13) der Kopfteil (6) des Knochenfixationsmittels (1) in den zwei Stabilisationsteilen (2;3) lösbar fixierbar ist.

25. Vorrichtung nach einem der Ansprüche 1 bis 24, **dadurch gekennzeichnet, dass** das im unteren Stabilisationsteil (3) angeordnete Klemmelement (10) durch erste Spannmittel (14') in der Kavität (8) des unteren Stabilisationsteiles (3) fixierbar ist und das im oberen Stabilisationsteil (2) angeordnete Klemmelement (9) durch zweite Spannmittel (14") in der Kavität (7) des oberen Stabilisationsteiles (2) fixierbar ist.

26. Vorrichtung nach einem der Ansprüche 1 bis 24, **dadurch gekennzeichnet, dass**:
D1) das obere Stabilisationsteil (2) eine Vertiefung (24) umfasst, die eine quer zur Zentralachse (4) stehende Längsachse (25) aufweist und aussen am Stabilisationsteil (2) offen ist, so dass ein stabförmiger Längsträger in die Vertiefung (24) einführbar ist; und das untere Stabilisationsteil (3) einen stabförmigen Fortsatz (28) umfasst, der eine zweite ebenfalls quer Zentralachse (4) stehende Längsachse (27) aufweist, wobei der Fortsatz (28) und die Vertiefung (24) komplementär ausgestaltet sind;
oder dass
D2) beide Stabilisationsteile (2;3) Vertiefungen (24) zur Aufnahme je eines Längsträgers (16) umfassen.

## Claims

1. Device for pivotably connecting two stabilization members (2, 3) that are parts of a fixation device for bone to a bone fixation means (1), comprising at least one bone fixation means (1) with a central axis (4), which has an anchoring section (5) for fixation in or on the bone and a head section (6) that is connectable to the two stabilization members (2, 3);
**characterized in that**
A) the device is provided with an upper and a lower stabilization member (2, 3) arranged axially behind each other, each with a cavity (7, 8) that penetrates the respective stabilization member (2, 3) coaxially to the central axis (4);
B) in each cavity (7, 8) a clamping element (9, 10) is provided and rotatably arranged, which is elastically deformable perpendicular to the central axis (4) and has a central bore hole (11, 12) running coaxially to the central axis (4); and
C) the device has at least one wedge or cone (13) axially jammable in the central bore holes (11,12), by means of which the head section (6) of the bone fixation means (1) is loosenably fixable in at least one of the stabilization members (2, 3).

2. Device according to claim 1 **characterized in that** the device additionally comprises a tensioning means (14) acting upon the head section (6) for axial movement of the at least one wedge or cone (13).

3. Device according to claim 1 or claim 2 **characterized in that** at least one wedge or cone (13) is one-piece with the head section (6) of the bone fixation means (1).

4. Device according to one of the claims 1 to 3 **characterized in that** at least one wedge or cone (13) is one-piece with the tensioning means (14).

5. Device according to one of the claims 1 to 3 **characterized in that** the device comprises a lower and an upper wedge or cone (13', 13"), wherein the lower wedge or cone (13') expands relative to the anchoring section (5) of the bone fixation means (1) and the upper wedge or cone (13") expands relative to the rear end (19) of the bone fixation means (1).

6. Device according to claim 5 **characterized in that** the upper wedge or cone (13") is one-piece with the tensioning means (14).

7. Device according to claim 5 or 6 **characterized in that** the lower wedge or cone (13') is one-piece with the head section (6) of the bone fixation means (1).

8. Device according to one of the claims 1 to 6 **characterized in that** the head section (6) of the bone fixation means (1) comprises terminally arranged a bolt (20) which is coaxial to the central axis (4) and has an external thread (21).

9. Device according to claim 8 **characterized in that** the tensioning means (14) is a nut (22) that can be screwed onto the external thread (21).

10. Device according to one of the claims 1 to 6 **characterized in that** the head section (6) of the bone fixation means (1) is provided at its rear end (19) with a bore hole which is coaxial to the central axis (4) and has an internal thread.

11. Device according to claim 10 **characterized in that** the tensioning means (14) is a screw which is screwable into the internal thread.

12. Device according to one of the claims 1 to 11 **characterized in that** an upper clamping element (9) is fixable by upper tensioning means (14") and a lower clamping element (10) is fixable by lower tensioning means (14').

13. Device according to claim 12 **characterized in that** the head section (6) comprises two cones (13', 13") separated by a threaded intermediate part (40), wherein the threaded intermediate part (40) is axially positioned relative to the lower clamping element (10) in such a way that the lower clamping element (10) is blockable by a tensioning means (14") positionable on the threaded intermediate part (40).

14. Device according to claim 12, **characterized in that**
a) the lower clamping element (10) rises above the head section (6);
b) the head section (6) is provided with a hole (38) with an internal thread (39) at the rear end (19) of the bone fixation means (1);
c) the lower tensioning means (14') comprises a threaded part (41) insertable into the internal thread (39) and an axially adjacent cone (42) becoming broader towards the threaded part (41), and
d) the cone (42) is complementary to the internal cone (13") to the clamping element (9).

15. Device according to claim 14 **characterized in that** the cone (42) of the lower tensioning means (14') ends at a threaded bolt which axially rises above the upper clamping element (9) onto which the upper tensioning means (14") provided with an internal thread can be screwed.

16. Device according to claim 14 **characterized in that** the upper clamping element (9) axially rises above the tapered end of the cone (42) of the lower tensioning means (14') and the tapered end of the cone (42) has a hole (45) with an internal thread (46) into which an upper tensioning means (14") provided with an external thread (47) and a head (48) can be screwed.

17. Device according to one of the claims 1 to 16 **characterized in that** at least one clamping element (9, 10) consists externally of several circular cylindrical segments (37) arranged axially behind each other.

18. Device according to one of the claims 1 to 16 **characterized in that** at least one cavity (7,8) is provided axially terminally with each a narrowing (35) with a circular edge (36) concentric to the central axis (4).

19. Device according to one of the claims 1 to 16 **characterized in that** the external surfaces of the clamping elements (9, 10) are roughened.

20. Device according to one of the claims 1 to 16 or 19 **characterized in that** the surfaces of the cavities (7, 8) are roughened.

21. Device according to one of the claims 1 to 16 **characterized in that** the external surfaces of the clamping elements (9, 10) comprise a three-dimensional structure.

22. Device according to one of the claims 1 to 16 or 21 **characterized in that** the surfaces of the cavities (7, 8) comprise a three-dimensional structure.

23. Device according to one of the claims 1 to 22 **characterized in that** at least one clamping element (9, 10) consists of a material that is softer than the material used for the walls of the corresponding cavity (7, 8).

24. Device according to one of the claims 1 to 23, **characterised in that** by means of the at least one wedge or cone (13) which is jammable in the central bore holes (11, 12) the head section (6) of the bone fixation means (1) is loosenably fixable in the two stabilization members (2, 3).

25. Device according to one of the claims 1 to 24, **characterised in that** the clamping element (10) arranged in the lower stabilization member (3) is fixable in the cavity (8) of the lower stabilization member (3) by means of first tensioning means (14') and the clamping element (9) arranged in the upper stabilization member (2) is fixable in the cavity (7) of the upper stabilization member (2) by means of second tensioning means (14").

26. Device according to one of the claims 1 to 24, **characterised in that**:
D1) the upper stabilization member (2) comprises a recess (24), which has a longitudinal axis (25) perpendicular to the central axis (4) and which is open at the outer side of the stabilization member (2), so that a bar shaped longitudinal carrier is insertable into the recess (24); and that lower stabilization member (3) comprises a bar shaped extension (28), which has a second longitudinal axis (27) perpendicular to the central axis (4) as well, wherein the extension (28) and the recess (24) are complementarily configured; or that
D2) both stabilisation members (2; 3) comprise recesses (24) for receiving a longitudinal carrier (16) each.

## Revendications

1. Dispositif destiné au raccordement pivotant de deux parties de stabilisation (2 ; 3) faisant partie d'un équipement de fixation pour os, avec un moyen de fixation osseuse (1), comprenant au moins un moyen de fixation osseuse (1) qui comprend un axe central (4) et qui présente une partie d'ancrage (5) destinée à être fixée dans ou sur l'os et une partie de tête (6) pouvant être raccordée aux deux parties de stabilisation (2 ; 3) ;
**caractérisé en ce que**
A) le dispositif présente, disposées axialement l'une derrière l'autre, une partie de stabilisation supérieure et une partie de stabilisation inférieure (2 ; 3) avec chacune une cavité (7 ; 8) qui traverse la partie de stabilisation (2 ; 3) correspondante de façon coaxiale à l'axe central (4) ;
B) dans chaque cavité (7 ; 8), il y a, disposé en rotation, un élément de coincement (9 ; 10) déformable élastiquement transversalement à l'axe central (4), avec un alésage central (11 ; 12) qui est coaxial à l'axe central (4) ; et
C) le dispositif comprend au moins un coin ou cône (13) pouvant être coincé axialement dans les alésages centraux (11 ; 12), ce qui fait que la partie de tête (6) du moyen de fixation osseuse (1) peut être fixée de façon détachable dans au moins une des parties de stabilisation (2 ; 3).

2. Dispositif selon la revendication 1, **caractérisé en ce qu'**il comprend en plus des moyens de serrage (14) agissant sur la partie de tête (6) et destinés au déplacement axial du coin ou cône (13) au moins au nombre de un.

3. Dispositif selon la revendication 1 ou 2, **caractérisé en ce qu'**au moins un coin ou cône (13) est d'un seul tenant avec la partie de tête (6) du moyen de fixation osseuse (1).

4. Dispositif selon une des revendications 1 à 3, **caractérisé en ce qu'**au moins un coin ou cône (13) est d'un seul tenant avec le moyen de serrage (14).

5. Dispositif selon une des revendications 1 à 4, **caractérisé en ce qu'**il comprend un coin ou cône inférieur ou supérieur (13' ; 13"), le coin ou cône inférieur (13') s'élargissant vers la partie d'ancrage (5) sur le moyen de fixation osseuse (1), et le coin ou cône supérieur (13") s'élargissant vers l'extrémité arrière (19) du moyen de fixation osseuse (1).

6. Dispositif selon la revendication 5, **caractérisé en ce que** le coin ou cône supérieur (13") est d'un seul tenant avec le moyen de serrage (14).

7. Dispositif selon la revendication 5 ou 6, **caractérisé en ce que** le coin ou cône inférieur (13') est d'un seul tenant avec la partie de tête (6) du moyen de fixation osseuse (1).

8. Dispositif selon une des revendications 1 à 6, **caractérisé en ce que** la partie de tête (6) du moyen de fixation osseuse (1) comprend, à une extrémité, un boulon (20) coaxial à l'axe central (4) et muni d'un filet extérieur (21).

9. Dispositif selon la revendication 8, **caractérisé en ce que** le moyen de serrage (14) est un écrou (22) qui peut être vissé par le biais du filet extérieur (21).

10. Dispositif selon une des revendications 1 à 6, **caractérisé en ce que** la partie de tête (6) du moyen de fixation osseuse (1) présente à son extrémité arrière (19) un alésage coaxial à l'axe central (4) et muni d'un filet intérieur.

11. Dispositif selon la revendication 10, **caractérisé en ce que** le moyen de serrage (14) est une vis qui peut se visser dans le filet intérieur.

12. Dispositif selon une des revendications 1 à 11, **caractérisé en ce qu'**un élément de coincement (9) supérieur peut être fixé par des éléments de serrage (14") supérieurs, et **en ce qu'**un élément de coincement (10) inférieur peut être fixé par des éléments de serrage (14') inférieurs.

13. Dispositif selon la revendication 12, **caractérisé en ce que** la partie de tête (6) comprend deux cônes (13' ; 13") qui sont séparés par une pièce intermédiaire filetée (40), la pièce intermédiaire filetée (40) étant positionnée axialement par rapport à l'élément de coincement inférieur (10) de telle façon que l'élément de coincement inférieur (10) peut être bloqué par un moyen de serrage (14') pouvant être placé sur la pièce intermédiaire filetée (40).

14. Dispositif selon la revendication 12, **caractérisé en ce que**
a) l'élément de coincement (10) inférieur dépasse axialement la partie de tête (6) ;
b) la partie de tête (6) présente, à l'extrémité arrière (19) du moyen de fixation osseuse (1), un alésage (38) muni d'un filet intérieur (39) ;
c) le moyen de serrage (14') inférieur comprend une pièce filetée (41) pouvant être introduire dans le filet intérieur (39), et un cône (42) axialement adjacent et s'élargissant vers la pièce filetée (41) ; et
d) le cône (42) est complémentaire au cône intérieur (13") dans l'élément de coincement (9).

15. Dispositif selon la revendication 14, **caractérisé en ce que** le cône (42) du moyen de serrage (14') inférieur se termine au niveau d'un boulon fileté qui dépasse axialement l'élément de coincement (9) supérieur et sur lequel l'élément de serrage (14") supérieur muni d'un filet intérieur peut être vissé.

16. Dispositif selon la revendication 14, **caractérisé en ce que** l'élément de coincement (9) supérieur dépasse axialement l'extrémité rétrécie du cône (42) du moyen de serrage (14') inférieur, et **en ce que** l'extrémité rétrécie du cône (42) présente un alésage (45) muni d'un filet intérieur (46) dans lequel peut être vissé un élément de serrage (14") supérieur muni d'un filet extérieur (47) et d'une tête (48).

17. Dispositif selon une des revendications 1 à 16, **caractérisé en ce qu'**au moins un élément de coincement (9; 10) se compose extérieurement de plusieurs segments (37) cylindriques à section circulaire disposés les uns derrière les autres axialement.

18. Dispositif selon une des revendications 1 à 16, **caractérisé en ce qu'**au moins une cavité (7 ; 8) présente, à une extrémité axialement, respectivement un resserrement (35) muni d'une arête (36) circulaire, concentrique à l'axe central (4).

19. Dispositif selon une des revendications 1 à 16, **caractérisé en ce que** les surfaces extérieures des parties de coincement (9 ; 10) sont munies de rugosités.

20. Dispositif selon une des revendications 1 à 16 ou 19, **caractérisé en ce que** les surfaces des cavités (7 ; 8) sont munies de rugosités.

21. Dispositif selon une des revendications 1 à 16, **caractérisé en ce que** les surfaces extérieures des parties de coincement (9 ; 10) comprennent une structuration tridimensionnelle.

22. Dispositif selon une des revendications 1 à 16 ou 21, **caractérisé en ce que** les surfaces des cavités (7 ; 8) comprennent une structuration tridimensionnelle.

23. Dispositif selon une des revendications 1 à 22, **caractérisé en ce qu'**au moins un élément de coincement (9 ; 10) est constitué d'un matériau qui est plus tendre comparé au matériau de paroi de la cavité (7 ; 8) correspondante.

24. Dispositif selon une des revendications 1 à 23, **caractérisé en ce que**, du fait du coin ou cône (13) au moins au nombre de un pouvant être coincé axialement dans les alésages centraux (11 ; 12), la partie de tête (6) du moyen de fixation osseuse (1) peut être fixée de façon détachable dans les deux parties de stabilisation (2 ; 3).

25. Dispositif selon une des revendications 1 à 24, **caractérisé en ce que** l'élément de coincement (10) disposé dans la partie de stabilisation (3) inférieure peut être fixé par des premiers moyens de serrage (14') dans la cavité (8) de la partie de stabilisation (3) inférieure, et **en ce que** l'élément de coincement (9) disposé dans la partie de stabilisation (2) supérieure peut être fixé par des deuxièmes éléments de serrage (14") dans la cavité (7) de la partie de stabilisation (2) supérieure.

26. Dispositif selon une des revendications 1 à 24, **caractérisé en ce que** :
D1) la partie de stabilisation (2) supérieure comprend un creux (24) qui présente un axe longitudinal (25) transversal à l'axe central (4) et qui est ouvert extérieurement sur la partie de stabilisation (2) de telle sorte qu'un support longitudinal en forme de barre peut être introduit dans le creux (24) ; et **en ce que** la partie de stabilisation (3) inférieure comprend un prolongement (28) en forme de barre qui présente un deuxième axe longitudinal (27) également transversal à l'axe central (4), le prolongement (28) et le creux (24) étant constitués de façon complémentaire ;
ou **en ce que**
D2) les deux parties de stabilisation (2 ; 3) comprennent des creux (24) pour loger respectivement un support longitudinal (16).
